# EUROPÄISCHE PATENTSCHRIFT

(11) **EP 1 501 816 B1**
(45) Veröffentlichungstag und Bekanntmachung des Hinweises auf die Patenterteilung: **09.05.2007**
(21) Anmeldenummer: 03717319.2
(22) Anmeldetag: 25.04.2003
(51) Int. Cl.: C07D 307/20

(54) **VERFAHREN ZUR HERSTELLUNG VON GAMMA-BUTYROLACTON**
METHOD FOR PRODUCING GAMMA-BUTYROLACTONE
PROCEDE DE PRODUCTION DE GAMMA-BUTYROLACTONE

(30) Priorität: 30.04.2002 DE 10219224
(43) Veröffentlichungstag der Anmeldung: 02.02.2005
(73) Patentinhaber: BASF Aktiengesellschaft, 67056 Ludwigshafen (DE)
(72) Erfinder: PINKOS, Rolf, 67098 Bad Dürkheim (DE); FISCHER, Rolf-Hartmuth, 69121 Heidelberg (DE); RÖSCH, Markus, 55276 Dienheim (DE); BOTTKE, Nils, 68165 Mannheim (DE); SCHLITTER, Stephan, 67117 Limburgerhof (DE); HESSE, Michael, 67549 Worms (DE); WECK, Alexander, 67251 Freinsheim (DE); WINDECKER, Gunther, 67067 Ludwigshafen (DE); HEYDRICH, Gunnar, 67117 Limburgerhof (DE)
(86) Internationale Anmeldenummer: PCT/EP2003/004287
(87) Internationale Veröffentlichungsnummer: WO 2003/093256

(56) Entgegenhaltungen:
- WO-A-02/47815
- WO-A-02/48130
- US-A- 5 122 495
- US-A- 5 347 021
- US-A- 5 698 713
- US-B1- 6 284 703
- US-B1- 6 297 389

## Beschreibung

Die vorliegende Erfindung betrifft ein Verfahren zur Herstellung von gegebenenfalls C₁- bis C₄-alkylsubstituiertem gamma-Butyrolacton durch katalytische Hydrierung von gegebenenfalls C₁- bis C₄-alkylsubstituierter Maleinsäure, Bernsteinsäure und/oder deren Estern und Anhydriden in der Gasphase. Unter Derivaten der Maleinsäure werden in der vorliegenden Anmeldung Ester und Anhydride verstanden, die ebenso wie die Säure einen oder mehrere C₁- bis C₄-Alkylsubstituenten aufweisen können. Es wird ein 10 bis 80 Gew.-% Kupferoxid und 10 bis 90 Gew.-% mindestens eines Katalysatorträgers aus der Gruppe Siliciumdioxid, Titandioxid, Hafniumdioxid, Magnesiumsilikat, Aktivkohle, Siliciumcarbid, Zirkondioxid und alpha-Aluminiumoxid enthaltender Katalysator verwendet, bei dem das Kupferoxid feinverteilt und innig vermischt mit den anderen Bestandteilen vorliegt.

Die Herstellung von gamma-Butyrolacton (GBL) durch Gasphasenhydrierung von Maleinsäureanhydrid (MSA) ist eine seit vielen Jahren bekannte Reaktion. Zur Durchführung dieser katalytischen Reaktion sind in der Literatur zahlreiche Katalysatorsysteme beschrieben.

Ein ausschließlich aus Cu- und Al-Oxiden aufgebauter Katalysator für die MSA-Gasphasenhydrierung zu GBL wird in der WO 97/24346 offenbart. Dieser Katalysator enthält 50 bis 95 %, vorzugsweise 80 bis 90 % Kupferoxid, 3 bis 30 Gew-%, vorzugsweise 5 bis 15 Gew.-% Aluminiumoxid sowie optional einen Binder. Bei der Hydrierung von MSA mit einem derartigen Katalysator bei einem Versuchszeitraum von 1600 Stunden betrug die GBL-Ausbeute 92 bis 93 Gew.-%. Die hohen Kupfergehalte der Katalysatoren beeinträchtigen die Wirtschaftlichkeit durch hohe Materialkosten.

Aus EP 404 808 sind Schalenkatalysatoren mit einer katalytischen aktiven Masse der allgemeinen Formel Cu₁Zn_{b}Al_{c}M_{d}Oₓ, in der M mindestens ein Element, das ausgewählt ist aus der Gruppe bestehend aus den Gruppen IIA bis IVA, Gruppe VIII, Ag, Au, Gruppen IIIB, VIIB und den seltenen Erden, zur Katalyse der MSA-Hydrierung zu Gemischen aus GBL und THF bekannt. Die katalytisch aktive Masse wird auf einen im wesentlichen inerten, mindestens teilweise porösen, eine äußere Oberfläche aufweisenden Träger aufgetragen. Im Gegensatz zum eingeträgerten Katalysator, der als Hauptprodukt THF liefert, entsteht hierbei GBL als bevorzugtes Produkt. Im Beispiel 3 wird beispielsweise mit einem Katalysator der Zusammensetzung CuAl_{1,2}ZnCr_{0,004}Oₓ bei einem Druck von 2 bar, 280°C, einem Wasserstoff MSA-Verhältnis von 100 und einer GHSV (Gas hourly space velocity) von 11420 h eine GBL-Ausbeute von 91,3 % erzielt. Nachteilig ist, dass auch hier sämtliche in den Beispielen verwendeten Katalysatoren Chrom enthalten. Weiterhin werden große Mengen an Bernsteinsäureanhydrid gebildet.

Chromfreie Katalysatorsysteme zur Herstellung von GBL durch MSA-Hydrierung sind beispielsweise aus WO 99/35139 bekannt. Die offenbarten, gegebenenfalls geträgerten Katalysatoren enthalten als katalytisch aktive Masse 50 bis 90 % CuO und 10 bis 50 % ZnO. Gemäß Beispiel 2 wird in Gegenwart eines 64 Gew.-% CuO und 23,5 Gew.-% ZnO enthaltenden Katalysators gearbeitet. Dabei werden bei 245 bis 270°C und 5 bar GBL-Ausbeuten von 94,9 bis 96,6 % erzielt.

Alle in den vorstehend genannten Druckschriften beschriebenen Katalysatoren weisen den Nachteil auf, dass sie noch unerwünschte Nebenprodukt liefern. Häufig enthalten die Katalysatoren auch Chrom. Zudem sind die erzielbaren Standzeiten der Katalysatoren unbefriedigend.

Die Aufgabe der vorliegenden Erfindung besteht darin, ein Verfahren bereitzustellen, mit dem gegebenenfalls auch C₁- bis C₄-alkylsubstituiertes GBL durch Hydrierung von gegebenenfalls C₁- bis C₄-alkylsubstituierter Maleinsäure, Bernsteinsäure und/oder deren Estern und Anhydriden hergestellt werden kann, wobei sowohl MSA als auch ihre Derivate einen oder mehrere C₁- bis C₄-Alkylsubstituenten aufweisen können sollen. Dieses Verfahren soll dabei kontinuierlich und mit chromfreien Katalysatoren betrieben werden können und möglichst große Mengen des Wertstoffs GBL liefern, um so eine größtmögliche Wirtschaftlichkeit zu erreichen. Weiterhin sollte der Katalysator mit MSA betrieben werden können, das nicht aufwendig vorgereinigt wurde und trotzdem eine hohe Standzeit aufweisen, also kein häufiges Regenerieren erfordern.

Diese Aufgabe wird gelöst durch ein Verfahren zur Herstellung von gamma-Butyrolacton (GBL) durch katalytische Hydrierung von gegebenenfalls C₁- bis C₄-alkylsubstituierter Maleinsäure, Bernsteinsäure und/oder deren Estern und Anhydriden mit einem chromfreien Katalysator, enthaltend 10 bis 80 Gew.-% Kupferoxid (CuO) und 10 bis 90 Gew.-% mindestens eines Katalysatorträgers aus der Gruppe Siliciumdioxid, Titandioxid, Hafniumdioxid, Magnesiumsilikat, Aktivkohle, Siliciumcarbid, alpha-Aluminiumoxid und Zirkondioxid, wobei das Kupferoxid feinverteilt und innig vermischt mit den anderen Bestandteilen vorliegt.

Das erfindungsgemäße Verfahren zeichnet sich dadurch aus, dass es kostengünstig betrieben werden kann und hohe GBL-Ausbeuten und Selektivitäten erreicht werden. Eine kontinuierliche Durchführungsweise ist möglich und erfindungsgemäß bevorzugt.

Ein wichtiger Aspekt der vorliegenden Erfindung ist die Wahl des Katalysators, der als katalytisch aktiven Hauptbestandteil Kupferoxid aufweist. Dieses Kupferoxid ist auf einem Träger aufgebracht, der eine geringe Anzahl saure Zentren aufweisen muß. Hohe Anzahlen saurer Zentren im Trägermaterial wie zum Beispiel im gamma-Aluminiumoxid, begünstigen die Bildung von Tetrahydrofuran als unerwünschtes Nebenprodukt. Der erfindungsgemäß verwendete Katalysator ist daher frei von saurem gamma-Aluminiumoxid. Geeignete Trägermaterialien mit geringer Anzahl an sauren Zentren sind Siliciumoxid, Titandioxid, Hafniumdioxid, Magnesiumsilikat, Aktivkohle und Siliciumcarbid oder deren Gemische, von denen Siliciumdioxid, Zirkondioxid, Titandioxid, Aktivkohle und alpha-Aluminiumoxid besonders bevorzugt sind.

Die Menge an Kupferoxid liegt bei Werten von < 80 Gew.-%. Bevorzugte Katalysatorzusammensetzungen weisen < 70 Gew.-% Kupferoxid und > 10 Gew.-% Träger, besonders bevorzugte Katalysatoren 10 bis 65 Gew.-% Kupferoxid und 35 bis 90 Gew.-% Träger auf. Niedrige Kupferoxid-Gehalte sind auch aufgrund des damit erzielten Kostenvorteils bevorzugt.

Optionsweise können die erfindungsgemäß verwendeten Katalysatoren, die chromfrei sind, 0 bis 10 Gew.-%, vorzugsweise bis 0,1 bis 5 Gew.-% eines oder mehrerer weiterer Metalle oder einer oder mehrerer Metallverbindungen der Elemente, bevorzugt ein Oxid, ausgewählt aus der Gruppe Silber, Molybdän, Wolfram, Rhodium, Mangan, Rhenium, Ruthenium, Palladium und Platin, von denen Mangan, Rhenium, Palladium und Silber bevorzugt enthalten sind, aufweisen.

Weiterhin können die erfindungsgemäß verwendeten Katalysatoren optional bis 50 Gew.-%, bevorzugt bis 20 Gew.-%, eines Metalls oder einer Metallverbindung, vorzugsweise eines Oxids, des Zinks, eines Elements der Gruppen 1 oder 2 (IA und IIA der alten IUPAC-Nomenklatur) des Periodensystems der Elemente und/oder der seltenen Erdmetalle einschließlich der Lanthaniden enthalten.

Bevorzugte Elemente, die in den erfindungsgemäßen Katalysatoren enthalten sein können, sind Zink, Lithium, Natrium, Kalium, Rubidium, Cäsium, Magnesium, Calcium, Strontium, Barium, Scandium, Yttrium, Lanthan und die seltenen Erdelemente der Ordnungszahl 58 bis 71. Besonders bevorzugt sind Natrium, Kalium, Calcium, Magnesium, Lanthan und Zink.

Die eingesetzten Katalysatoren können zudem ein Hilfsmittel in einer Menge von 0 bis 10 Gew.-% enthalten. Unter Hilfsmittel versteht man organische und anorganische Stoffe, die zu einer verbesserten Verarbeitung während der Katalysatorherstellung und/ oder zu einer Erhöhung der mechanischen Festigkeit der Katalysatorformkörper beitragen. Derartige Hilfsmittel sind dem Fachmann bekannt; Beispiele umfassen Graphit, Stearinsäure, Kieselgel und/oder Kupferpulver.

Die Katalysatoren lassen sich nach dem Fachmann bekannten Methoden herstellen. Bevorzugt sind Verfahren, bei denen das Kupferoxid fein verteilt und innig vermischt mit den anderen Bestandteilen anfällt.

Diese Ausgangsmaterialien können nach bekannten Methoden zu den Formkörpern verarbeitet werden, beispielsweise Extrudieren, Tablettieren oder durch Agglomerationsverfahren, gegebenenfalls unter Zusatz von Hilfsmitteln.

Erfindungsgemäße Katalysatoren können beispielsweise auch durch Aufbringen der Aktivkomponenten auf einen Träger hergestellt werden, beispielsweise durch Tränken oder Aufdampfen. Weiterhin können erfindungsgemäße Katalysatoren durch Verformen einer heterogenen Mischung aus Aktivkomponente oder Vorläuferverbindung hiervon mit einer Trägerkomponente oder Vorläuferverbindung hiervon erhalten werden.

Bei der erfindungsgemäßen Hydrierung, bei der neben MSA andere, nachstehend definierte C₄-Dicarbonsäuren oder deren Derivate als Edukt eingesetzt werden können, wird der Katalysator in reduzierter, aktivierter Form verwendet. Die Aktivierung erfolgt mit reduzierenden Gasen, vorzugsweise Wasserstoff oder Wasserstoff/Inertgas-Gemischen, entweder vor oder nach dem Einbau in den Reaktor, in dem das erfindungsgemäße Verfahren durchgeführt wird. Wurde der Katalysator in oxidischer Form in den Reaktor eingebaut, so kann die Aktivierung sowohl vor dem Anfahren der Anlage mit der erfindungsgemäßen Hydrierung als auch während des Anfahrens, also in situ, durchgeführt werden. Die separate Aktivierung vor dem Anfahren der Anlage erfolgt im allgemeinen mit reduzierenden Gasen, vorzugsweise Wasserstoff oder Wasserstoff/Inertgas-Gemischen bei erhöhten Temperaturen, vorzugsweise zwischen 100 und 300°C. Bei der sogenannten in-situ-Aktivierung erfolgt die Aktivierung beim Hochfahren der Anlage durch Kontakt mit Wasserstoff bei erhöhter Temperatur.

Die Katalysatoren werden als Formkörper verwendet. Beispiele umfassen Stränge, Rippstränge, andere Extrudatformen, Tabletten, Ringe, Kugeln und Splitt.

Die BET-Oberfläche der Kupferkatalysatoren beträgt im oxidischen Zustand 10 bis 400 m2/g, vorzugsweise 15 bis 200 m2/g, insbesondere 20 bis 150 m2/g. Die Kupferoberfläche (N₂O-Zersetzung) des reduzierten Katalysators beträgt im Einbauzustand > 0,2 m2/g, vorzugsweise > 1 m2/g, insbesondere > 2 m²/g.

Gemäß einer Variante der Erfindung werden Katalysatoren verwendet, die eine definierte Porosität aufweisen. Diese Katalysatoren zeigen als Formkörper ein Porenvolumen von ≥0,01 ml/g für Porendurchmesser > 50 nm, vorzugsweise ≥ 0,025 ml/g für Porendurchmesser > 100 nm und insbesondere ≥ 0,05 ml/g für Porendurchmesser > 200 nm. Weiterhin liegt das Verhältnis von Makroporen mit einem Durchmesser > 50 nm zum Gesamtporenvolumen für Poren mit einem Durchmesser > 4 nm bei Werten > 10 %, bevorzugt > 20 %, insbesondere > 30 %. Die erwähnten Porositäten wurden durch Quecksilber-Intrusion nach DIN 66133 bestimmt. Es wurden die Daten im Porendurchmesserbereich von 4 nm bis 300 nm ausgewertet.

Die erfindungsgemäß verwendeten Katalysatoren besitzen im allgemeinen eine ausreichende Standzeit. Für den Fall, dass die Aktivität und/oder Selektivität des Katalysators dennoch im Laufe ihrer Betriebszeit sinken sollte, kann diese durch dem Fachmann bekannte Maßnahmen regeneriert werden. Hierzu zählt vorzugsweise eine reduktive Behandlung des Katalysators im Wasserstoffstrom bei erhöhter Temperatur. Gegebenenfalls kann der reduktiven Behandlung eine oxidative vorausgehen. Hierbei wird die Katalysatorschüttung mit einem molekularen Sauerstoff enthaltenden Gasgemisch, beispielsweise Luft, bei erhöhter Temperatur durchströmt. Weiterhin besteht die Möglichkeit, den Katalysator mit einem geeigneten Lösungsmittel, beispielsweise Ethanol, THF oder GBL, zu waschen und anschließend in einem Gasstrom zu trocknen.

Unter dem Begriff Maleinsäure und deren Derivate als Ausgangsmaterialien werden im Bezug auf die vorliegende Anmeldung Maleinsäure und Bernsteinsäure, die gegebenenfalls einen oder mehrere C₁-C₄-Alkylsubstituenten aufweisen sowie die Ester und Anhydride dieser gegebenenfalls alkylsubstituierten Säuren verstanden. Ein Beispiel einer solchen Säure ist Citraconsäure. Vorzugsweise werden die jeweiligen Anhydride einer gegebenen Säure eingesetzt. Insbesondere ist das verwendete Edukt MSA.

Das erfindungsgemäße Verfahren zeichnet sich dadurch aus, dass es kostengünstig betrieben werden kann, hohe GBL-Ausbeuten von bis zu 95 % und mehr erreicht, und durch einfache Aufarbeitung reines GBL liefert.

Das Verfahren kann diskontinuierlich oder kontinuierlich, bevorzugt kontinuierlich, durchgeführt werden.

Zum Erzielen der erfindungsgemäßen GBL-Ausbeuten ist weiterhin das Einhalten gewisser Reaktionsparameter erforderlich.

Ein wichtiger Parameter ist das Einhalten einer geeigneten Reaktionstemperatur. Dies wird zum einen erreicht durch eine genügend hohe Eingangstemperatur der Edukte. Diese liegt bei Werten von > 180°C bis 300°C, insbesondere > 220 bis 300°C, vorzugsweise 240 bis 290°C. Um eine akzeptable bzw. hohe GBL-Selektivität und - Ausbeute zu erhalten, muss die Reaktion so durchgeführt werden, dass am Katalysatorbett, an dem die eigentliche Reaktion stattfindet, eine geeignet hohe Reaktionstemperatur herrscht. Diese sogenannte Hot-Spot-Temperatur wird nach dem Eintritt der Edukte in den Reaktor eingestellt und liegt bei Werten von 240 bis 320°C, vorzugsweise 240 bis 300°C.

Das Wasserstoff/Edukt-Molverhältnis ist ebenfalls ein Parameter, der einen wichtigen Einfluss auf die Produktverteilung und auch die Wirtschaftlichkeit des erfindungsgemäßen Verfahrens hat. Aus wirtschaftlicher Sicht ist ein niedriges Wasserstoff/Edukt-Verhältnis wünschenswert. Die Untergrenze liegt bei einem Wert von rund 3, wobei jedoch generell höhere Wasserstoff/Edukt-Molverhältnisse von 20 bis 600 angewendet werden. Der Einsatz der oben beschriebenen, erfindungsgemäßen Katalysatoren sowie das Einhalten der oben beschriebenen Temperaturwerte erlaubt den Einsatz günstiger, niedriger Wasserstoff/Edukt-Verhältnisse, die vorzugsweise bei Werten von 20 bis 200, vorzugsweise 40 bis 150 liegen. Der günstigste Bereich liegt bei Werten von 50 bis 100. Die bei der Reaktion entstehende Wärme wird in diesem Fall durch eine innere Wärmeabfuhr abgeführt. Alternativ kann auch mit Wasserstoff: MSA-Verhältnissen von 400:1 bis 600:1 gearbeitet werden. In diesem Fall wird die Wärme mit dem Kreisgas aus dem Reaktor ausgetragen. Es kann in diesem Fall ein kostengünstiger Schachtreaktor verwendet werden.

Um die erfindungsgemäß verwendeten Wasserstoff/Edukt-Molverhältnisse einzustellen, wird ein Teil, vorteilhafterweise die Hauptmenge, des Wasserstoffs im Kreis gefahren. Hierzu setzt man im allgemeinen die dem Fachmann bekannten Kreisgasverdichter ein. Die chemisch durch die Hydrierung verbrauchte Wasserstoffmenge wird ergänzt. In einer bevorzugten Ausführungsform wird ein Teil des Kreisgases ausgeschleust, um Inertverbindungen, beispielsweise n-Butan, zu entfernen. Der im Kreis geführte Wasserstoff kann auch, gegebenenfalls nach Vorheizen, zum Verdampfen des Eduktstroms benutzt werden.

Auch der Volumenstrom der Reaktionsgase, generell ausgedrückt als GHSV (Gas Hourly Space Veloaty) ist eine wichtige Größe des erfindungsgemäßen Verfahrens. Die GHSV-Werte des erfindungsgemäßen Verfahrens liegen bei Werten von 100 bis 20.000 Nm³/m³h, vorzugsweise 1000 bis 3000 Nm³/m³h, insbesondere 1100 bis 2500 Nm³/m³h.

Der Druck, bei dem die erfindungsgemäße Hydrierung durchgeführt wird, liegt bei Werten von 1 bis 30 bar, vorzugsweise 1 bis 15 bar, insbesondere 1 bis 10 bar.

Gemeinsam mit dem Wasserstoff-Kreisgas werden alle Produkte im Kreis geführt, die beim Kühlen des aus dem Hydrierreaktor austretenden Gasstroms nicht oder nicht vollständig auskondensieren. Dies sind vor allem Wasser und Nebenprodukte wie THF, Methan und Butan. Die Kühltemperatur beträgt 0 bis 60 °C, vorzugsweise 20 bis 45°C. Der THF-Gehalt des Kreisgases beträgt 0,1 bis 5 Vol.-%, insbesondere 0,5 bis 3 Vol.-%.

Bei der Hydrierung von MSA zu GBL wird das Zwischenprodukt Bernsteinsäureanhydrid (BSA) durchlaufen. Das erfindungsgemäße Verfahren macht es möglich, MSA und Derivate zu Reaktionsgemischen zu hydrieren, die nur wenig THF und entsprechend 95 Mol-% GBL (bezogen auf MSA) und mehr enthalten. Die GBL-Ausbeute lässt sich vor allem durch die Wahl des Hydrierkatalysators, die Katalysator-Belastung, die Hydriertemperatur, den Reaktionsdruck und das Ausgangsprodukt/Wasserstoff-Molverhältnis beeinflussen. Die THF-Menge steigt mit zunehmender Anzahl an sauren Katalysator-Zentren, mit abnehmender Katalysator-Belastung, steigender Temperatur und steigendem Reaktionsdruck.

Als Reaktortypen kommen alle, für heterogen katalysierte Reaktionen mit einem gasförmigen Edukt- und Produktstrom geeigneten Apparate in Betracht. Bevorzugt sind Rohrreaktoren, Schachtreaktoren oder Reaktoren mit innerer Wärmeabfuhr, beispielsweise Rohrbündelreaktoren, es ist auch der Einsatz eines Wirbelbetts möglich. Bevorzugt eingesetzt werden Rohrbündelreaktoren.

Der aus dem Reaktor austretende Gasstrom wird auf 10 bis 100°C gekühlt. Dabei werden die Reaktionsprodukte auskondensiert und in einen Abscheider geleitet. Der nicht-kondensierte Gasstrom wird vom Abscheider abgezogen und dem Kreisgasverdichter zugeführt. Eine kleine Kreisgasmenge wird ausgeschleust. Die auskondensierten Reaktionsprodukte werden dem System kontinuierlich entnommen und der Aufarbeitung zugeführt. Als Nebenprodukt findet man in der auskondensierten Flüssigkeitsphase hauptsächlich Wasser und n-Butanol neben geringen Mengen an Propanol, Methanol, Ethanol, n-Butyraldehyd, Butylmethylether und weiteren Sauerstoff enthaltenden Verbindungen, deren Konzentration unter 200 ppm liegt.

Das erfindungsgemäße Verfahren zeichnet sich dadurch aus, dass zu hydrierende Edukte unterschiedlicher Reinheit in die Hydrierreaktion eingesetzt werden können. Selbstverständlich kann ein Edukt hoher Reinheit, insbesondere MSA, in die Hydrierreaktion eingesetzt werden. Der erfindungsgemäß verwendete Katalysator sowie die sonstigen erfindungsgemäß gewählten Reaktionsbedingungen ermöglichen aber auch den Einsatz von Edukten, insbesondere MSA, die mit den üblichen, bei der Oxidation von Benzol, Butenen oder n-Butan anfallenden Verbindungen sowie eventuell weiteren Komponenten verunreinigt sind. Somit kann das erfindungsgemäße Hydrierverfahren in einer weiteren Ausführungsform eine vorgeschaltete Stufe umfassen, die das Herstellen des zu hydrierenden Edukts durch partielle Oxidation eines geeigneten Kohlenwasserstoffs sowie das Abtrennen des zu hydrierenden Edukts aus dem damit erhaltenen Produktstrom umfasst.

Insbesondere ist dieses zu hydrierende Edukt MSA. Dabei wird bevorzugt MSA eingesetzt, welches aus der Partialoxidation von Kohlenwasserstoffen stammt. Geeignete Kohlenwasserstoffströme sind Benzol, C₄-Olefine (z.B. n-Butene, C₄-Raffinatströme) oder n-Butan. Besonders bevorzugt eingesetzt wird n-Butan, da es einen preiswerten, wirtschaftlichen Einsatzstoff darstellt. Verfahren zur Partialoxidation von n-Butan sind beispielsweise in Ullmann's Encyclopedia of Industrial Chemistry, 6^{th} Edition, Electronic Release, Maleic and Fumaric Acids - Maleic Anhydride beschrieben.

Der so erhaltene Reaktionsaustrag wird dann in einem geeigneten organischen Lösungsmittel oder -Gemisch aufgenommen, das bei Atmosphärendruck einen um mindestens 30°C höheren Siedepunkt als MSA hat.

Dieses Lösungsmittel (Absorptionsmittel) wird auf eine Temperatur im Bereich zwischen 20 und 160°C, bevorzugt zwischen 30 und 80°C, gebracht. Der Maleinsäureanhydrid enthaltende Gasstrom aus der Partialoxidation kann in vielfältiger Weise mit dem Lösungsmittel in Kontakt gebracht werden: (i) Einleiten des Gasstroms in das Lösungsmittel (z. B. über Gaseinleitungsdüsen oder Begasungsringe), (ii) Einsprühen des Lösungsmittels in den Gasstrom und (iii) Gegenstromkontakt zwischen dem nach oben strömenden Gasstrom und dem nach unten strömenden Lösungsmittel in einer Boden-oder Packungskolonne. In allen drei Varianten können die dem Fachmann bekannten Apparate zur Gasabsorption eingesetzt werden. Bei der Wahl des einzusetzenden Lösungsmittels ist darauf zu achten, dass dies nicht mit dem Edukt, beispielsweise dem vorzugsweise eingesetzten MSA, reagiert. Geeignete Lösungsmittel sind: Trikresylphosphat, Dibutylmaleat und Dibutylfumarat, Dimethylmaleat und Dimethylfumarat, hochmolekulare Wachse, aromatische Kohlenwasserstoffe mit einem Molekulargewicht zwischen 150 und 400 und einem Siedepunkt oberhalb 140°C, wie beispielsweise Dibenzylbenzol; Dialkylphtahalte mit C₁-C₈-Alkylgruppen, beispielsweise Dimethylphthalat, Diethylphthalat, Dibutylphthalat, Di-n-Propyl-und Di-iso-Propyl-phthalat; Di-C₁-C₄-Alkylester anderer aromatischer und aliphatischer Dicarbonsäuren, beispielsweise Dimethyl-2,3-Naphthalin-Dicarbonsäure, Dimethyl-1,4-Cyclohexan-Dicarbonsäure, Methylester langkettiger Fettsäuren mit beispielsweise 14 bis 30 Kohlenstoffatomen, hochsiedende Ether, beispielsweise Dimethylether von Polyethylenglykol, beispielsweise Tetraethylenglykoldimethylether. Anstelle von Diestern können auch die jeweiligen Monoester eingesetzt werden. Bevorzugte Beispiele sind Maleinsäure- und Fumarsäuremonoalkylester, insbesondere Methyl- und Butylester.

Der Einsatz von Phthalaten ist bevorzugt.

Die nach der Behandlung mit dem Absorptionsmittel resultierende Lösung hat generell einen MSA-Gehalt von etwa 5 bis 400 Gramm pro Liter.

Der nach der Behandlung mit dem Absorptionsmittel verbleibende Abgasstrom enthält hauptsächlich die Nebenprodukte der vorangegangenen Partialoxidation, wie Wasser, Kohlenmonoxid, Kohlendioxid, nicht umgesetzte Butane, Essig- und Acrylsäure. Der Abgasstrom ist praktisch frei von MSA.

Anschließend wird das gelöste MSA aus dem Absorptionsmittel ausgetrieben. Dies erfolgt mit Wasserstoff bei oder maximal 10 % oberhalb des Druckes der anschließenden Hydrierung oder alternativ im Vakuum mit anschließender Kondensation von verbleibendem MSA. In der Strippkolonne wird ein Temperaturprofil beobachtet, das sich aus den Siedepunkten von MSA am Kopf und dem nahezu MSAfreien Absorptionsmittel am Sumpf der Kolonne bei dem jeweiligen Kolonnendruck und der eingestellten Verdünnung mit Trägergas (im ersten Fall mit Wasserstoff) ergibt.

Um Verluste an Lösungsmittel zu verhindern, können sich oberhalb der Zufuhr des Roh-MSA-Stromes Rektifiziereinbauten befinden. Das vom Sumpf abgezogene, nahezu MSA-freie Absorptionsmittel wird wieder der Absorptionszone zugeführt. Im Fall der Direktstrippung mit Wasserstoff wird vom Kopf der Kolonne ein nahezu gesättigter Gasstrom von MSA in Wasserstoff abgezogen. Im anderen Fall wird das kondensierte MSA in einen Verdampfer gepumpt und dort in den Kreisgasstrom verdampft.

Der MSA-Wasserstoff-Strom enthält noch Nebenprodukte, die bei der partiellen Oxidation von n-Butan, Butenen oder Benzol mit Sauerstoff enthaltenden Gasen entstehen, sowie nicht abgetrenntes Absorptionsmittel. Hierbei handelt es sich vor allem um Essigsäure und Acrylsäure als Nebenprodukte, Wasser, Maleinsäure sowie die vorzugsweise als Absorptionsmittel verwendeten Dialkylphthalate. Das MSA enthält Essigsäure in Mengen von 0,01 bis 1 Gew.-%, vorzugsweise 0,1 bis 0,8 Gew.-% und Acrylsäure in Mengen von 0,01 bis 1 Gew.-%, vorzugsweise 0,1 bis 0,8 Gew.-%, bezogen auf MSA.
In der Hydrierstufe werden Essigsäure und Acrylsäure ganz oder teilweise zu Ethanol bzw. Propanol hydriert. Der Maleinsäure-Gehalt beträgt 0,01 bis 1 Gew.-%, insbesondere 0,05 bis 0,3 Gew.-%, bezogen auf MSA.

Werden Dialkylphthalate als Absorptionsmittel eingesetzt, hängt deren Gehalt im MSA stark vom richtigen Betrieb der Strippkolonne, insbesondere vom Verstärkungsteil ab. Phthalatgehalte von bis 1,0 Gew.-%, insbesondere bis 0,5 Gew.-% sollten bei geeigneter Betriebsweise nicht überschritten werden, da sonst der Verbrauch an Absorptionsmittel zu hoch wird.

Der so erhaltene Wasserstoff/Maleinsäureanhydrid-Strom wird nun der Hydrierzone zugeführt und wie oben beschrieben hydriert. Die Katalysatoraktivität und -standzeit ist dabei verglichen mit dem Einsatz von stark, beispielsweise durch Destillation vorgereinigtem MSA praktisch unverändert.

Der auskondensierte Hydrieraustrag wird nun destillativ zu spezifikationsgerechtem GBL aufgearbeitet.

### Beispiele

### Beispiel 1

### a) Katalysatorherstellung

### Katalysator A:

Katalysator A wurde hergestellt durch Tränkung von 5 mm Strängen einer Mischung aus 92 Gew.-% SiO₂ und 8 % CaO mit einer Lösung von Kupfercarbonat in konzentriertem wässrigem Ammoniak. Die Tränkung erfolgte durch Aufdüsen der Kupfercarbonatlösung während 15 min.

Die getränkten Stränge wurden 3 h bei 120°C getrocknet und danach 2 h bei 350°C calziniert. Der fertige Katalysator A enthält 13 Gew.-% CuO, 7 % CaO und 80 % SiO₂ (berechnet auf 100 % Oxide).

### Katalysator B:

Katalysator B wurde hergestellt durch Tränkung von SiO₂-Kugeln mit einem Durchmesser von 3 bis 5 mm mit einer Lösung von Kupfercarbonat in konzentriertem wässrigen Ammoniak. Die Tränkung erfolgte in überstehender Lösung während 15 Minuten. Die getränkten Kugeln wurden 5 h bei 120°C getrocknet und danach 2 h bei 250°C calciniert. Diese Tränk- und Kalzinierschritte wurden mehrmals wiederholt. Der fertige Katalysator B enthält 25,6 Gew.-% CuO und 74,4 % SiO₂ (berechnet auf 100 % Oxide).

### Katalysator B

(25 Gew.-% CuO, 75 Gew.-% SiO₂) wurde entsprechend hergestellt.

### b) Katalysator-Aktivierung

Vor dem Reaktionsbeginn wurde der Katalysator in der Hydrierapparatur einer Wasserstoffbehandlung unterzogen. Hierzu wurde der Reaktor auf 240 bzw. 265°C temperiert und der Katalysator die in Tabelle 1 angegebene Zeit mit dem jeweils angegebenen Gemisch aus Wasserstoff und Stickstoff bei Atmosphärendruck aktiviert.

**Tabelle 1:**

| Zeit (Minuten) | Temperatur [°C] | Wasserstoff (Nl/h) | Stickstoff (Nl/h) |
|---|---|---|---|
| 15 | 240 | 500 | 10 |
| 10 | 240 | 500 | 20 |
| 20 | 240 | 400 | 40 |
| 120 | 240 | 200 | 65 |
| 720 | 265 | 0 | 65 |

### c) Hydrierapparatur

Die zur Hydrierung verwendete Druckapparatur bestand aus einem Verdampfer, einem Reaktor, einem Kühler mit Quenchzulauf, einer Wasserstoffzufuhr, einer Abgasleitung und einem Kreisgasgebläse. Der Druck in der Apparatur wurde konstant gehalten.

Das aufgeschmolzene MSA wurde von oben auf den vorgeheizten (245°C) Verdampfer gepumpt und verdampft. Auf den Verdampfer gelangt ebenfalls von oben eine Mischung aus frischem Wasserstoff und Kreisgas. Wasserstoff und MSA gelangten so von unten in den temperierten Reaktor. Der Reaktorinhalt bestand aus einem Gemisch aus Glasringen und Katalysator. Nach der Hydrierung verließ das entstandene GBL zusammen mit Wasser, anderen Reaktionsprodukten und Wasserstoff den Reaktor und wird im Kühler durch Quenchen niedergeschlagen. Ein Teil des Kreisgases wurde ausgeschleust, bevor der Rest, mit Frischwasserstoff vermischt, wieder in den Verdampfereintritt.

Der kondensierte flüssige Reaktionsaustrag, das Abgas und das Kreisgas wurden gaschromatographisch quantitativ analysiert.

### Beispiele 2 und 3

### Hydrierung von aus n-Butan hergestelltem Maleinsäureanhydrid

Der Reaktor der in Beispiel 1c beschriebenen Hydrierapparatur wurde mit 220 ml des nach Beispiel 1 a hergestellten Katalysators A und 126 ml Raschigringen gefüllt. Die Aktivierung erfolgte wie in Beispiel 1 b beschrieben.

Als Edukt wurde aus n-Butan hergestelltes Maleinsäureanhydrid eingesetzt. Dabei wurde während der Umsetzung keinerlei Desaktivierung des Katalysators, d.h. kein Rückgang des Maleinsäureanhydrid-Umsatzes und/oder der gamma-Butyrolacton-Ausbeute beobachtet. In Tabelle 2 sind die Reaktionsparameter der Hydrierung und die Ergebnisse zusammengefaßt.

### Beispiel 4

Anstelle von Katalysator A wurde Katalysator B mit der Zusammensetzung 25 Gew.-% CuO, 74,4 Gew.-% Sio₂ verwendet. Auch er wurde nach Aktivierung des Katalysators aus n-Butan hergestelltem Maleinsäureanhydrid ohne Desaktivierung während der Umsetzung betrieben. In Tabelle 2 sind die Reaktionsparameter der Hydrierung und die Ergebnisse zusammengefasst.

**Tabelle 2:**

| Bsp | Katalysator | Temperatur [°C] | Druck [bar] | Belastung [kg_{MSA}/Kgₖₐₜh] | H₂/MSA [mol/mol] | Umsatz [%] | GBL [%] | BDO [%] | THF [%] | BSA [%] |
|---|---|---|---|---|---|---|---|---|---|---|
| 2 | A | 280 | 5,4 | 0,15 | 115 | 100 | 94 | 0,3 | 1 | 0 |
| 3 | A | 267 | 15 | 0,1 | 126 | 100 | 84 | 0,7 | 6,2 | 0,2 |
| 4 | B | 260 | 5,3 | 0,1 | 93 | 100 | 93 | 0,4 | 1,3 | 0 |

| | | | | | | | | | | |
|---|---|---|---|---|---|---|---|---|---|---|
| BDO = 1,4-Butandiol BSA = Bernsteinsäureanhydrid | | | | | | | | | | |

## Patentansprüche

1. Verfahren zur Herstellung von gegebenenfalls C₁- bis C₄-alkyl-substituiertem gamma-Butyrolacton durch katalytische Hydrierung von gegebenenfalls C₁- bis C₄-alkylsubstituierter Maleinsäure, Bernsteinsäure und/oder deren Estern und Anhydriden mit einem chromfreien Katalysator enthaltend 10 bis 80 Gew.-% Kupferoxid, bis 50 Gew.-% mindestens eines Metalls oder einer Metallverbindung der Elemente der Gruppen 1 oder 2 des Periodensystems der Elemente und/oder der seltenen Erdmetalle einschließlich der Lanthaniden und 10 bis 90 Gew.-% mindestens eines Katalysatorträgers aus der Gruppe Siliciumdioxid, Titandioxid, Hafniumdioxid, Magnesiumsilikat, Aktivkohle, Siliciumcarbid, Zirkondioxid und alpha-Aluminiumoxid, wobei das Kupferoxid feinverteilt und innig vermischt mit den anderen Bestandteilen vorliegt.

2. Verfahren nach Anspruch 1, **dadurch gekennzeichnet, dass** der Katalysator bis 10 Gew.-% eines oder mehrerer weiterer Metalle oder deren Verbindungen aus der Gruppe Silber, Molybdän, Wolfram, Mangan, Rhodium, Ruthenium, Rhenium, Palladium und Platin aufweist.

3. Verfahren nach einem der Ansprüche 1 oder 2, **dadurch gekennzeichnet, dass** dieses bei Drücken von 1 bis 30 bar, durchgeführt wird.

4. Verfahren nach einem der Ansprüche 1 bis 3, **dadurch gekennzeichnet, dass** das Molverhältnis Wasserstoff/Edukt bei Werten von 20 bis 600 liegt.

5. Verfahren nach einem der Ansprüche 1 bis 4, **dadurch gekennzeichnet, dass** die GHSV bei Werten von 100 bis 20.000 Nm³/m³h liegt.

6. Verfahren nach einem der Ansprüche 1 bis 5, **dadurch gekennzeichnet, dass** die Eingangstemperatur bei Werten von > 180 bis 300°C liegt.

7. Verfahren nach einem der Ansprüche 1 bis 6, **dadurch gekennzeichnet, dass** der Katalysator vor oder nach dem Einbau in den Reaktor und vor dem Einsatz in die Hydrierreaktion durch Reduktion aktiviert wird, vorzugsweise durch Behandlung mit Wasserstoff oder einem Wasserstoff/Inertgas-Gemisch.

8. Verfahren nach einem der Ansprüche 1 bis 7, **dadurch gekennzeichnet, dass** der Katalysator ein Hilfsmittel in einer Menge < 10 Gew.-%, vorzugsweise Graphit, Stearinsäure, Kieselgel und/oder Kupferpulver, enthält.

9. Verfahren nach einem der Ansprüche 1 bis 8, **dadurch gekennzeichnet, dass** der Katalysator-Formkörper ein Porenvolumen von ≥ 0,01 ml/g für Porendurchmesser > 50 nm aufweist.

10. Verfahren nach einem der Ansprüche 1 bis 9, **dadurch gekennzeichnet, dass** in dem Katalysator-Formkörper das Verhältnis von Makroporen mit einem Durchmesser > 50 nm zum Gesamtporenvolumen für Poren mit einem Durchmesser > 4 nm bei Werten > 10 % liegt.

11. Verfahren nach einem der Ansprüche 1 bis 10, **dadurch gekennzeichnet, dass** Maleinsäureanhydrid eingesetzt wird, das durch Oxidation von Benzol, C₄-Olefinen oder n-Butan hergestellt wurde, wobei das durch Oxidation erhaltene Roh-Maleinsäureanhydrid mit einem Lösungsmittel aus dem Rohproduktgemisch extrahiert und anschließend aus diesem Lösungsmittel mit Wasserstoff ausgetrieben wurde.

## Claims

1. A process for preparing unsubstituted or C₁-C₄-alkyl-substituted gamma-butyrolactone by catalytic hydrogenation of unsubstituted or C₁-C₄-alkyl-substituted maleic acid, succinic acid and/or esters and anhydrides in the presence of chromium-free catalyst comprising from 10 to 80% by weight of copper oxide, up to 50% by weight of at least one metal or metal compound of elements of groups 1 and 2 of the Periodic Table of the Elements and/or the rare earth metals including the lanthanides and from 10 to 90% by weight of at least one catalyst support selected from the group consisting of silicon dioxide, titanium dioxide, hafnium dioxide, magnesium silicate, activated carbon, silicon carbide, zirconium dioxide and alpha-aluminum oxide, the copper oxide being finely divided and intimately mixed with the other constituents.

2. The process according to claim 1 wherein the catalyst further comprises up to 10% by weight of one or more further metals or compounds thereof selected from the group consisting of silver, molybdenum, tungsten, manganese, rhodium, ruthenium, rhenium, palladium and platinum.

3. The process according to claim 1 or 2 carried out at pressures of from 1 to 30 bar.

4. The process according to any of claims 1 to 3 wherein the molar ratio of hydrogen/starting material is in the range from 20 to 600.

5. The process according to any of claims 1 to 4 wherein the GHSV is in the range from 100 to 20,000 standard m³/m³h.

6. The process according to any of claims 1 to 5 wherein the inlet temperature is in the range from > 180 to 300°C.

7. The process according to any of claims 1 to 6 wherein the catalyst is activated by reduction, preferably by treatment with hydrogen or a hydrogen/inert gas mixture, before or after it is installed in the reactor and before it is used in the hydrogenation reaction.

8. The process according to any of claims 1 to 7 wherein the catalyst contains an auxiliary, preferably graphite, stearic acid, silica gel and/or copper powder, in an amount of < 10% by weight.

9. The process according to any of claims 1 to 8 wherein the shaped catalyst body has a pore volume of ≥ 0.01 ml/g for pore diameters of > 50 nm.

10. The process according to any of claims 1 to 9 wherein the ratio of macropores having a diameter of > 50 nm to the total pore volume for pores having a diameter of > 4 nm in the shaped catalyst body is > 10%.

11. The process according to any of claims 1 to 10 wherein maleic anhydride which has been prepared by oxidation of benzene, C₄-olefins or n-butane is used, where the crude maleic anhydride obtained by oxidation has been extracted from the crude product mixture by means of a solvent and has subsequently been stripped from this solvent by means of hydrogen.

## Revendications

1. Procédé de préparation de gamma-butyrolactone éventuellement substitué par de l'alkyle en C₁-C₄ par hydrogénation catalytique d'acide maléique éventuellement substitué par de l'alkyle en C₁-C₄, d'acide succinique et/ou ses esters et anhydrides avec un catalyseur exempt de chrome contenant 10 à 80 % en poids d'oxyde de cuivre, jusqu'à 50 % en poids d'au moins un métal ou un composé métallique des éléments des groupes 1 ou 2 du système périodique des éléments et/ou des métaux des terres rares incluant les lanthanides et 10 à 90 % en poids d'au moins un support de catalyseur issu du groupe dioxyde de silicium, dioxyde de titane, dioxyde d'hafnium, silicate de magnésium, charbon actif, carbure de silicium, dioxyde de zircon et oxyde d'alpha-aluminium, l'oxyde de cuivre étant présent de manière finement dispersée et de manière intimement mélangée avec les autres composants.

2. Procédé selon la revendication 1, **caractérisé en ce que** le catalyseur présente jusqu'à 10 % en poids d'un ou de plusieurs autres métaux ou de leurs composés issus du groupe argent, molybdène, tungstène, manganèse, rhodium, ruthénium, rhénium, palladium et platine.

3. Procédé selon l'une quelconque des revendications 1 ou 2, **caractérisé en ce que** celui-ci est mis en oeuvre à des pressions de 1 à 30 bars.

4. Procédé selon l'une quelconque des revendications 1 à 3, **caractérisé en ce que** le rapport molaire hydrogène/éduit se situe à des valeurs de 20 à 600.

5. Procédé selon l'une quelconque des revendications 1 à 4, **caractérisé en ce que** la GHSV (vélocité spatiale horaire de gaz) se situe à des valeurs de 100 à 20 000 Nm³/m³h.

6. Procédé selon l'une quelconque des revendications 1 à 5, **caractérisé en ce que** la température d'entrée se situe à des valeurs de plus de 180 jusqu'à 300°C.

7. Procédé selon l'une quelconque des revendications 1 à 6, **caractérisé en ce que** le catalyseur est activé par réduction avant ou après l'installation dans le réacteur et avant l'utilisation dans la réaction d'hydrogénation, de manière préférée par traitement avec de l'hydrogène ou un mélange hydrogène/gaz inerte.

8. Procédé selon l'une quelconque des revendications 1 à 7, **caractérisé en ce que** le catalyseur contient un adjuvant en une quantité < 10 % en poids, de manière préférée du graphite, de l'acide stéarique, du gel de silice et/ou de la poudre de cuivre.

9. Procédé selon l'une quelconque des revendications 1 à 8, **caractérisé en ce que** le corps moulé formant catalyseur présente un volume poreux ≥ 0,01 ml/g pour un diamètre de pore > 50 nm.

10. Procédé selon l'une quelconque des revendications 1 à 9, **caractérisé en ce que** le rapport des macropores ayant un diamètre > 50 nm sur le volume poreux total des pores ayant un diamètre > 4 nm se situe à des valeurs > 10 % dans le corps moulé formant catalyseur.

11. Procédé selon l'une quelconque des revendications 1 à 10, **caractérisé en ce que** de l'anhydride d'acide maléique préparé par oxydation de benzène, d'oléfines en C₄ ou de n-butane est utilisé, l'anhydride d'acide maléique brut obtenu par oxydation étant extrait du mélange de produit brut avec un solvant et étant pour finir expulsé de ce solvant avec de l'hydrogène.
